# EUROPÄISCHE PATENTSCHRIFT

(11) **EP 1 125 588 B1**
(45) Veröffentlichungstag und Bekanntmachung des Hinweises auf die Patenterteilung: **03.01.2007**
(21) Anmeldenummer: 01103026.9
(22) Anmeldetag: 09.02.2001
(51) Int. Cl.: A61L 9/22, F24F 3/16, F24F 11/00

(54) **Verfahren und Vorrichtung zur Luftbehandlung wenigstens eines Raumes durch Luftionisation**
Method and device for air ionisation of at least one room
Procédé et dispositif pour l'ionisation de l'air d'au moins une pièce

(30) Priorität: 18.02.2000 DE 10007523
(43) Veröffentlichungstag der Anmeldung: 22.08.2001
(73) Patentinhaber: LK Luftqualität AG, 6014 Littau (CH)
(72) Erfinder: Fleischer, Werner, 6103 Schwarzenberg (CH)
(74) Vertreter: Kailuweit, Frank

(56) Entgegenhaltungen:
- EP-A- 0 616 175
- US-A- 5 259 553
- US-A- 5 759 487
- PATENT ABSTRACTS OF JAPAN vol. 2000, no. 15, 6. April 2001 (2001-04-06) -& JP 2000 337681 A (LK LUFTQUALITAET AG), 8. Dezember 2000 (2000-12-08)
- PATENT ABSTRACTS OF JAPAN vol. 1996, no. 02, 29. Februar 1996 (1996-02-29) -& JP 07 275736 A (BROTHER IND LTD), 24. Oktober 1995 (1995-10-24)

## Beschreibung

Die Erfindung betrifft Verfahren und Vorrichtungen zur Luftbehandlung wenigstens eines Raumes durch Luftionisation, wobei diese Ionisation durch elektrische Entladung in Ionisationsröhren oder in Koronaentladungsröhren erfolgt.

Bekannt ist, dass mit Ionisierungsapparaten die Raum- und damit die Atemluft behandelt werden kann. Dabei werden Bakterien und andere Keime abgetötet und Großmoleküle in kleinmolekulare Fragmente aufgespalten. Komplexe und große Moleküle sind unter anderem Geruchsstoffe, so dass mit einer Luftionisation eine Geruchsbelastung unterdrückt werden kann. Weiterhin können sowohl sogar gesundheitlich schädliche Lastsituationen in der Raumluft eliminiert als auch Mikroorganismen in der Luft wirksam reduziert werden.
In Ionisationsapparaten wird ein hohes elektrisches Feld zwischen zwei Spannungspotenzialen ausgenutzt. Dazu werden bekannterweise Ionisierungsröhren in Form von Glasröhren eingesetzt, bei denen koaxial die Innenseite beschichtet und die Außenseite elektrisch leitend sind. Wird eine ausreichend hohe elektrische Spannung angelegt, bildet das Glas der Wandung ein Dielektrikum, in dem ein großes elektrisches Feld vorhanden ist. Die durchströmende Luft wird mit Ionen angereichert. Ein wesentlicher Nachteil besteht darin, dass es ab einer bestimmten Spannung zu einer Bildung von Ozon kommt, die sich mit der Vergrößerung der Spannung erhöht.
In der DE 43 34 956.0 C2 sind ein Verfahren zur Luftbehandlung mit Ionen und eine Vorrichtung zur Durchführung des Verfahrens beschrieben, wobei die Langzeitstabilität eines Ionisationsapparates erhöht wird. Dabei wird die Entladungsspannung so gesteuert, dass die Schwelle zu einer erhöhten Ozonerzeugung stets unterschritten bleibt. Wie bei unbelasteter natürlicher Luft, wird technisch mit dem vorbeschriebenen Verfahren und der vorbeschriebenen Vorrichtung eine Mindestintensität an Sauerstoffionen von etwa 5 % als unterem Prozessgrenzwert eingehalten, das dem natürlichen Wert weitestgehend entspricht. Mittels der verwendeten Sensoren, in Form eines Luftqualitätssensors, eines Luftströmungsfühlers und eines Luftfeuchtefühlers kann die Einhaltung dieser Mindestintensität in einem Lastbereich im Wesentlichen eingehalten werden.
Beim Auftreten äußerer Störquellen, nämlich z.B. einer erhöhten Ozonbelastung in der Außenluft, beispielsweise bei Smog durch Sonneneinstrahlung, verschiedenen Situationen in der Natur, z.B. einer Inversionswetterlage, Gewitter, äußere Energiefelder, oder beim Auftreten innerer Störquellen, beispielsweise in der Nähe der Zuluftzuführung installierte elektrische Geräte zur Transformation von Spannungen oder Frequenzen, elektromagnetische Abstrahlungen oder anderer Strahlen kann die Belastung an Ozon in der Zuluft in unerwünschtem Maß ansteigen und zu einer Grenzwertüberschreitung führen.

Der in den Patentansprüchen 1 und 8 angegebenen Erfindung liegt das Problem zugrunde, ein Verfahren und eine Vorrichtung zur Durchführung des Verfahrens der eingangs genannten Gattung so weiterzubilden, dass eine Belastung an Ozon einen bestimmten Grenzwert nicht übersteigt.

Dieses Problem wird mit den in den Patentansprüchen 1 und 8 aufgeführten Merkmalen gelöst.

Die Verfahren und Vorrichtungen zur Luftbehandlung wenigstens eines Raumes durch Ionisation, wobei diese durch elektrische Entladung in Ionisationsröhren oder durch Koronaentladungen erfolgt, zeichnen sich insbesondere dadurch aus, dass die Höhe der Ionisationsleistung von einem elektrischen Steuergerät abhängig von durch Sensoren, nämlich einem ersten Luftqualitätssensor, einem Luftströmungsfühler, einem Luftfeuchtefühler, einem Ozonsensor und einem zweiten Luftqualitätssensor ermittelten Werten der oxidierbaren Luftbestandteile (z.B. vaporous organic compounds - VOC), der relativen Luftfeuchte, der Strömungsgeschwindigkeit/Volumenstrom, der Belastung an Ozon der zu behandelnden Luft unter Sicherstellung einer Mindestintensität an positiven und negativen Sauerstoffionen (adäquat des Luftzustandes in der Natur) bestimmt wird. Das erfindungsgemäße Verfahren wird dazu so geführt, dass insbesondere die Belastung der Außenluft mit flüchtigem Kohlenwasserstoff mit einem ersten Luftqualitätssensor, die Strömungsgeschwindigkeit oder der Volumenstrom der zu behandelnden Luft mit einem Luftströmungsfühler, die relative Luftfeuchte in der zu behandelnden Luft mit einem Luftfeuchtefühler, der Gehalt an Ozon in der Zuluft mit einem Ozonsensor und die oxidierbaren Luftbestandteile der Abluft oder der Umluft mit dem zweiten Luftqualitätssensors in der Umluftleitung zwischen dem Raum und dem Luftaufbereitungsgerät oder gegebenenfalls in der Abluftleitung oder gegebenenfalls im Raum direkt gemessen werden und entsprechend der Werte der Messungen die Höhe der Ionisationsleistung mindestens eines oder mehrerer Ionisationsapparate mit einem elektronischen Steuergerät so geregelt wird, dass die Mindestintensität an Sauerstoffionen und bei Auftreten eines zu hohen Wertes an Ozon dieses durch die Bildung freier Radikale als auch natürlicher Sauerstoffcluster zurückgeführt wird.
Der besondere Vorteil liegt dabei darin, dass insbesondere auch der Wert des Ozons in der Zuluft entsprechend bewertet, kontrolliert und bei Erreichen/Überschreiten von Fixpunkten Signale zum elektronischen Steuergerät gesendet werden. Dadurch wird der Ionisationsapparat so beeinflusst, dass eine schädliche Wirkung auf im Raum sich aufhaltende Personen weitestgehend vermieden wird. Das basiert auf dem Ozonsensor in der Zuluftleitung des Raumes, der über das elektronische Steuergerät mit dem Ionisationsapparat verbunden ist.

Das elektronische Steuergerät gewährleistet eine tatsächlich stabile und der Natur adäquate Zuluftionisation, wobei ein vorgegebener Ozongrenzwert nicht überschritten wird sowie bei einer Extremsituation Ozon eliminiert werden kann. Dabei werden die oben aufgezeigten Sensorsignale im Steuergerät so ausgewertet und aufbereitet, dass optimierte Wechselimpulse, die zu dem mindestens einen Ionisationsapparat geleitet werden, eine situationsgerechte Ionisationsintensität automatisch einstellen. Jeder Wechselimpuls ist eine volle Sinuskurve, die im Nulldurchgang im Bedarfsfall angeschnitten werden. Die Frequenz und Spannung werden dabei nicht verändert. Vorteilhafterweise werden gegenüber bisherigen Lösungen mehrere Wechselimpulse (mehrere Sinuskurven) zu Paketen oder Mengen zusammengefasst. Die Paketgröße und damit die Anzahl der Wechselimpulse je Paket oder Menge stellt eine Möglichkeit dar, um die Luftionisation weiterhin zu optimieren und gleichzeitig die Belastung des elektrischen Netzes zu minimieren. Es ist wesentlich, dass die Entladungsspannung dabei konstant bleibt, so dass eine stabile Luftionisation gewährleistet wird.
Die erfindungsgemäße Lösung zeichnet sich damit weiterhin durch die Gewährleistung einer Prozesssicherheit und eine Erhöhung des Wirkungsgrades aus, wobei auch die sichere Anwendung von lufttechnischen Anlagen mit einer gezielten Umluftnutzung gegeben ist. Damit werden bei hohen Außentemperaturen im Sommer und bei niedrigen Außentemperaturen im Winter erheblich Energiekosten für das Kühlen oder Heizen eingespart. Weitere vorteilhafte Effekte lassen sich bei der Rekonstruktion bestehender und Konzipierung neuer lufttechnischer Anlagen ableiten. Das sind unter anderem die Senkung des Anteils an Außenluft insbesondere bei einem hohen Anteil an Feuchtigkeit in der Außenluft oder die Erhöhung der Standzeiten der Luftfilter.

Die Ionisation der Zuluft und gegebenenfalls zusätzlich der Umluft führt vorteihafterweise dazu, dass insbesondere gasförmige flüchtige Kohlenwasserstoffe abgebaut, das Oxidationspotenzial der Luft gesenkt und Mikroorganismen eliminiert werden.

Vorteilhafte Ausgestaltungen der Erfindung sind in den Patentansprüchen 2 bis 7, 9 und 10 angegeben.

Ein Verfahren nach der Weiterbildung des Patentanspruchs 2 führt zur Schaffung einer natürlichen Behaglichkeit der Luft in einem Raum, wobei insbesondere gasförmige flüchtige Kohlenwasserstoffe abgebaut, das Oxidationspotenzial der Luft gesenkt und Mikroorganismen eliminiert werden.

Nach der Weiterbildung des Patentanspruchs 3 wird durch die Verfahren eine Mindestintensität an Sauerstoffionen etwa gleich 5% der installierten Ionisationskapazität gewährleistet. Dies entspricht den natürlichen Verhältnissen.

Die Ionisationsleistung des wenigstens einen Ionisationsapparates wird nach der Weiterbildung des Patentanspruchs 4 so gesteuert, dass diese bei sich erhöhenden Anteilen an flüchtigen Kohlenwasserstoffen und/oder Steigerung der Luftgeschwindigkeit und/oder Steigerung der relativen Luftfeuchte und/oder erhöhenden Anteil an oxidierbaren Luftbestandteilen steigt. Damit ist sichergestellt, dass sich bei verschlechternden Eigenschaften der Luftqualität im Raum weitestgehend unbelastete Zuluft durch den vorgegebenen Luftwechsel und der optimierten Ionisationsintensität in den Raum oder in die Aufenthaltszone gelangt.

Eine günstige Steuerung des Ionisationsapparates ist vorteilhafterweise nach den Weiterbildungen der Patentansprüche 5 und 9 über eine zeitlich anliegende periodische Wechselspannung gegeben. Dabei wird der Ionisationsapparat mit Wechselimpulsen oder zu Paketen zusammengefassten Wechselimpulsen einer zur Verfügung stehenden periodischen Wechselspannung beaufschlagt. Die optimierte Entladungsspannung ist dabei konstant.

Durch die Weiterbildung der Patentansprüche 6 und 10 wird der Anteil an Ozon so gesenkt, dass die gewünschten und vorgegebenen Grenzwerte eines Komfortraumklimas gewährleistet werden. In einem ersten Bereich wird dazu die Leistung des Ionisationsapparates gesenkt. Steigt der Wert des Ozongehaltes der Zuluft trotz der Absenkung der Luftionisation, so ist mindestens eine externe Ozonquelle vorhanden. In diesem Fall wird automatisch ein Modus zum Abbau des Ozones durch das elektronische Steuergerät geschaltet. Werden die vorgegebenen Grenzen wieder erreicht, wird die Vorrichtung wieder auf Normalbetrieb geschaltet. Im Modus "Ozonabbau" wird das Energieniveau des Ozons so verändert, dass es zerfällt. Die Fixpunkte zur Signalisierung von bestimmten Ozonwerten werden so gewählt, dass genügend Reaktionssicherheit vorhanden ist.

Das Vorhandensein einer ständigen Ionisationsintensität nach der Weiterbildung des Patentanspruchs 7 führt dazu, dass die dem Raum zuströmende Luft ständig beeinflusst wird. Bei sich plötzlich ändernden Bedingungen, z.B. durch viele Raucher im Raum oder stark wirkenden Reinigungsmitteln, ist die Zeitkonstante bis zu einer Reaktion dadurch wesentlich geringer, so dass die Raumluft positiv schneller beeinflusst oder sofort neutralisiert wird.

Ein Ausführungsbeispiel der Erfindung ist in den Zeichnungen dargestellt und wird im folgenden näher beschrieben.
Es zeigen:
Fig. 1 eine prinzipielle und schematische Darstellung einer Vorrichtung zur Luftbehandlung wenigstens eines Raumes durch Ionisation und
Fig. 2 eine prinzipielle Darstellung eines Paketes von zwei Wechselimpulsen zur Steuerung eines Ionisationsapparates.

Ein Verfahren und eine Vorrichtung zur Luftbehandlung wenigstens eines Raumes 9 durch Ionisation und damit mit Ionen werden nachfolgend in diesem Ausführungsbeispiel zusammen näher erläutert.

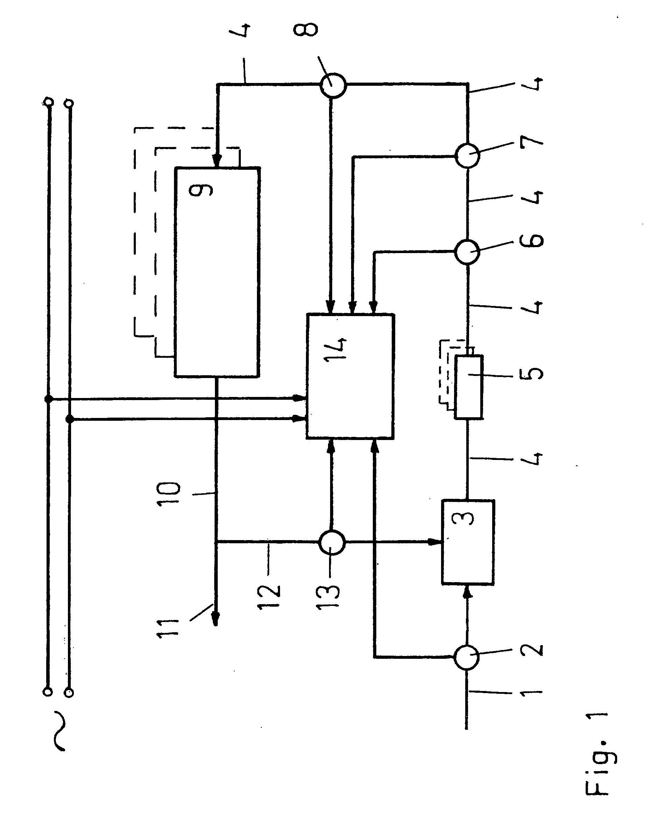

**Fig. 1** zeigt eine prinzipielle und schematische Darstellung einer Vorrichtung zur Luftbehandlung wenigstens eines Raumes durch Luftionisation. Ziel der Erfindung ist es, den Raum 9 mit behaglicher und den Erfordernissen entsprechenden Raumluft zu versorgen. Dementsprechend endet eine Zuluftleitung 4 in diesem Raum 9.

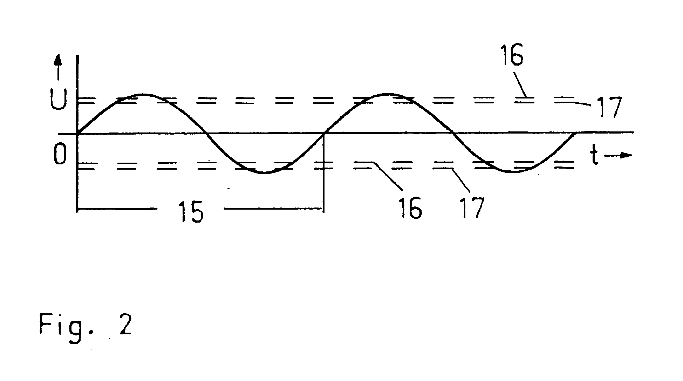

Die Zuluftleitung 4 kommt von einem Luftaufbereitungsgerät 3, an das eine Außenluftleitung 1 und die von dem Raum 9 kommende Umluftleitung 12 angeschlossen sind.
Die Vorrichtung umfasst auch ein elektronisches Steuergerät 14, das seine elektrische Energie über Leitungen aus einem elektrischen Energienetz bezieht. Diese Energiequelle ist dann freigeschaltet, wenn das Luftaufbereitungsgerät 3 in Funktion ist, wobei der Zuluftventilator den Zuluftstrom fördert.
Das elektronische Steuergerät 14 steuert über eine Steuerleitung mindestens einen Ionisationsapparat 5, der in die von dem Luftaufbereitungsgerät 3 kommende und zu dem Raum 9 führende Zuluftleitung 4 eingeschaltet ist. Hierzu erhält das elektrische Steuergerät 14 Informationen in Form von elektrischen Signalen von
- einem ersten Luftqualitätssensor 2, der die dem Luftaufbereitungsgerät 3 zuströmende Außenluftqualität berücksichtigt, insbesondere die Belastung der Außenluft mit flüchtigen Kohlenwasserstoffen - vaporous organic compounds (VOC) - oder dem eigentlichen Oxidationspotenzial der Außenluft,
- einem zweiten Luftqualitätssensor 13, der in die von dem Raum 9 kommende und zu dem Luftaufbereitungsgerät 3 führende Umluftleitung 12 oder gegebenenfalls in der Abluftleitung 10 oder gegebenenfalls im Raum 9 eingeschaltet ist und ebenfalls die flüchtigen oxidierbaren Raumluftbestandteile detektiert,
- einem Luftströmungsfühler 6, der die Strömungsgeschwindigkeit und damit die Luftfördermenge misst,
- einem Luftfeuchtefühler 7 und
- einem Ozonsensor 8.
   Der Luftströmungsfühler 6, der Luftfeuchtefühler 7 und der Ozonsensor 8 sind in die von dem Luftaufbereitungsgerät 3 kommende und zu dem Raum 9 führende Zuluftleitung 4 eingeschaltet.
   Der Luftströmungsfühler 6 stellt die Strömungsgeschwindigkeit in der Zuluftleitung 4 fest und der Luftfeuchtefühler 7 ermittelt die relative Luftfeuchte in der Zuluftleitung 4.
   In die Zuluftleitung 4 ist ferner ein Ozonsensor 8 eingeschaltet, der die Belastung an Ozon in der Zuluft feststellt und dieser Belastung äquivalente elektrische Signale dem elektronischen Steuergerät 14 zuführt.
   Die dem Ionisationsapparat 5 vom elektronischen Steuergerät 14 zugeführte elektrische Leistung wird in Abhängigkeit der Werte des ersten Luftqualitätssensors 2, des Luftströmungsfühlers 6, des Luftfeuchtefühlers 7, des Ozonsensors 8 und des zweiten Luftqualitätssensors 13 eingestellt. Dazu werden im elektronischen Steuergerät 14 die Signale von dem ersten Luftqualitätssensor 2, dem Luftströmungsfühler 6, dem Luftfeuchtefühler 7, dem Ozonsensor 8 und dem zweiten Luftqualitätssensors 13 so miteinander verknüpft, dass das elektronische Steuergerät 14 eine situationsgerechte Leistung in Form von Wechselimpulsraten oder mehreren zu Paketen oder Mengen zusammengefassten Wechselimpulsraten an den Ionisationsapparat 5 abgibt, wenn eine höhere Luftmenge und/oder ein größere relative Luftfeuchte und/oder eine größere Raumluftbelastung mit VOC auftritt oder auftreten. In diesen Fällen erfolgt eine Vergrößerung der Wechselimpulsrate oder der Anzahl zu Paketen zusammengefasster Wechselimpulsraten. Im positiven Extremfall, z.B. bei keinen Raumluftbelastungen, wird trotzdem eine minimale Ionisationsintensität auf den Ionisationsapparat 5 geschaltet.
   Im elektronischen Steuergerät 14 erfolgt dazu
- eine Gewichtung der einzelnen Parameter und eine Verknüpfung als Summe der einzelnen Vektoren,
- eine Verknüpfung als Produkt aus den einzelnen Beträgen oder
- eine andere mathematische Behandlung,
   so dass der Ionisationsapparat 5 mit einer entsprechenden optimalen oder erwünschten Leistung betrieben wird.
   Der Ionisationsapparat 5 wird mit zeitlichen Folgen einer periodischen Wechselspannung gleicher oder annähernd gleicher Amplitude betrieben. Die kleinste Einheit der Folge ist dabei eine Periode der periodischen Wechselspannung als ein Wechselimpuls 15 (Darstellung in der **Fig. 2**). Nichtbenötigte Perioden der periodischen Wechselspannung werden abgeführt. Dadurch wird gewährleistet, dass die Spannung bei der Entladung konstant bleibt und die für den Gesamtprozess bedeutenden funktionellen Daten sowohl stabil als auch steuerbar sind. Die periodische Wechselspannung besitzt dabei eine Frequenz, die der jeweils bereitgestellten Netzfrequenz entspricht. Ein Frequenzwandler ist nicht notwendig.
   Eine stabile Luftionisation und damit eine optimale Wirkungsweise, dass heißt ein hoher Anteil von positiv und negativ geladenen Sauerstoffionen mit einem hohen Bindungsbestreben - z.B. mit dem VOC-Anteil der Luft und mit einem minimalen Anteil von Radikalen in der Luft, wird nur mit einer definierten Entladungsspannung erzeugt. Diese muss weitestgehend konstant gehalten werden, so dass ein minimales Toleranzfeld eingehalten wird. Mit der Darstellung in der Fig. 2 wird nachfolgend das Verhalten der Koronaentladung beim Ändern der Entladungsspannung beim Überschreiten der Grenze 16 und Unterschreiten der Grenze 17 des Toleranzfeldes zwischen den Grenzen 16 und 17 einer optimalen Entladungsspannung beschrieben. Wird die Grenze 17 durch Anheben der Spannung des Ionisationsapparates 5 überschritten, wird progressiv die Ozonbelastung in der Zuluft ansteigen. Wird die Entladungsspannung dagegen unterhalb der Grenze 17 gesenkt, so ergibt sich ein Arbeitsfeld der Luftionisation, welche durch eine spontane koronare Entladung (Puffereffekt) gekennzeichnet ist, wobei ebenfalls unerwünschte Sauerstoffradikale oder Ozon freigesetzt werden. Erfindungsgemäß wird daher eine definierte Entladungsspannung im Prozess konstant gehalten. Eine situationsgerechte und stabile Luftionisation wird durch eine entsprechende Aktivierung der im Nulldurchgang angeschnittenen Sinuskurve der definierten Wechselspannung erzielt. Dabei ist eine derartige Sinuskurve ein jeweiliger Wechselimpuls 15, der den Ionisationsapparat 5 in Funktion setzt. Zur weiteren Optimierung der Wirkungsweise der Luftionisation ist das elektronische Steuergerät 14 so ausgelegt, dass zusätzlich die Wechselimpulsraten zu sinnvollen Paketen oder Mengen bestimmter Anzahl von Wechselimpulsen 15 zusammengefasst werden können.
   Die Signale des Ozonsensors werden wie folgt ausgewertet oder im Prozess genutzt:

- von 0 bis 0,06 ppm Ozon-Anteil im Zuluftstrom keine Einflussnahme,
- größer/gleich 0,06 ppm Ozon-Anteil Absenkung der momentanen Ionisationsleistung auf 50 %,
- bei weiterem Anstieg des Ozon-Anteils liegt eine externe Ozonquelle vor und es wird die beschriebene Maßnahme zum Abbau des Ozons eingeleitet.

Der Betrieb der erfindungsgemäßen Vorrichtung erfolgt weiterhin so, dass eine Mindest-Ionisationsleistung aufrechterhalten wird, auch wenn extrem niedrige Prozessdaten vorliegen. Das ist insbesondere dann der Fall, wenn der erste Luftqualitätssensor 2, der Luftströmungsfühler 6, der Luftfeuchtefühler 7, der Ozonsensor 8 und der zweite Luftqualitätssensor 13 dem elektronischen Steuergerät 14 signalisieren, dass an sich keine Ionisation erfolgen müsste. Dabei wird dem adäquaten Natureffekt entsprochen.

Insgesamt dient das Verfahren und die Vorrichtung der Schaffung einer natürlichen Behaglichkeit der Raumluft.

Während des Betriebs der Vorrichtung wird über die Abluftleitung 11 nur eine geringe Menge an Abluft abgeführt, der eine entsprechende Menge an Außenluft gegenübersteht, die dann über die Außenluftleitung 1 zugeführt wird. Damit ist eine gezielte Nutzung der Umluft zum Zweck der Energieeinsparung erreichbar.

### Bezugszeichenliste

- 1: Außenluftleitung
- 2: erster Luftqualitätssensor
- 3: Luftaufbereitungsgerät
- 4: Zuluftleitung
- 5: Ionisationsapparat
- 6: Luftströmungsfühler
- 7: Luftfeuchtefühler
- 8: Ozonsensor
- 9: Raum
- 10: Abluftleitung des Raumes
- 11: Fortluft
- 12: Umluftleitung
- 13: zweiter Luftqualitätssensor
- 14: elektronisches Steuergerät
- 15: ein Wechselimpuls = eine Periode
- 16: obere Grenze
- 17: untere Grenze

## Patentansprüche

1. Verfahren zur Luftbehandlung wenigstens eines Raumes durch Luftionisation, wobei diese Ionisation durch elektrische Entladung in Ionisationsröhren oder in Koronaentladungsröhren erfolgt, wobei die Höhe der Ionisationsleistung von einem elektronischen Steuergerät (14) abhängig von durch Sensoren, nämlich einem ersten Luftqualitätssensor (2), einem Luftströmungsfühler (6), einem Luftfeuchtefühler (7), einem Ozonsensor (8) und einem zweiten Luftqualitätssensor (13) ermittelten Werten
- oxidierbarer Luftbestandteile in der zu behandelnden Luft,
- relativer Luftfeuchte in der zu behandelnden Luft,
- Strömungsgeschwindigkeit oder Volumenstrom der zu behandelnden Luft,
- Ozonlast in der Zuluft und
- Mindestintensität an Sauerstoffionen
bestimmt wird.

2. Verfahren nach Patentanspruch 1, **dadurch gekennzeichnet, dass**
- insbesondere die Belastung der Außenluft mit flüchtigem Kohlenwasserstoff mit einem ersten Luftqualitätssensor (2),
- die Strömungsgeschwindigkeit oder der Volumenstrom der zu behandelnden Luft mit einem Luftströmungsfühler (6),
- die relative Luftfeuchte der zu behandelnden Luft mit einem Luftfeuchtefühler (7),
- der Gehalt an Ozon in der Zuluft und/oder der zu behandelnden Luft mit einem Ozonsensor (8) und
- die oxidierbaren Luftbestandteile des Raumes (9) oder der Abluft des Raumes (9) einer Abluftleitung (10) oder der Umluft in einer Umluftleitung (12) zwischen einer Abluftleitung (10) und einem Luftaufbereitungsgerät (3) mit dem zweiten Luftqualitätssensor (13) gemessen werden, und
entsprechend der Werte der Messungen die Höhe der Ionisationsleistung mindestens eines oder mehrerer Ionisationsapparate (5) mit einem elektronischen Steuergerät (14) so geregelt wird, dass die Mindestintensität an Sauerstoffionen und bei Auftreten eines zu hohen Wertes an Ozon der Ozonwert durch Aufspaltung zurückgeführt wird.

3. Verfahren nach Patentanspruch 1 oder 2, **dadurch gekennzeichnet, dass** eine Mindestintensität an Sauerstoffionen von etwa 5% der installierten Ionisationskapazität gewährleistet ist.

4. Verfahren nach jeweils einem der Patentansprüche 1 bis 3, **dadurch gekennzeichnet, dass** die Ionisationsleistung durch logische Verknüpfung der gemessenen Werte bei einem höheren Anteil an flüchtigen Kohlenwasserstoffen und/oder der Luftgeschwindigkeit und/oder der relativen Luftfeuchte und/oder oxidierbaren Luftbestandteilen steigt.

5. Verfahren nach jeweils einem der Patentansprüche 1 bis 4, **dadurch gekennzeichnet, dass** der Ionisationsapparat (5) über eine zeitlich anliegende periodische Wechselspannung als mindestens ein Wechselimpuls (15) oder als wenigstens ein Paket mit einer bestimmten Folge von Wechselimpulsen (15) steuerbar ist.

6. Verfahren nach jeweils einem der Patentansprüche 1 bis 5, **dadurch gekennzeichnet, dass** bei einem Gehalt an Ozon in der Zuluftleitung (4) von größer/gleich 0,06 ppm die Leistung des Ionisationsapparates (5) gesenkt wird und dass bei weiteren Anstieg des Wertes an Ozon die Zeit der anliegenden periodischen Wechselspannung als Wechselimpulse (15) und/oder Pakete mit Wechselimpulsen (15) bestimmter Anzahl so geändert wird, dass im über das elektronische Steuergerät (14) mit dem Ozonsensor (8) verbundenen Ionisationsapparat (5) eine Aufspaltung des Ozones eingeleitet wird.

7. Verfahren nach jeweils einem der Patentansprüche 1 bis 6, **dadurch gekennzeichnet, dass** bei niedrigen und keine Änderung des elektronischen Steuergerätes (14) hervorrufenden Prozessdaten eine Ionisationsintensität vorhanden ist.

8. Vorrichtung zur Luftbehandlung wenigstens eines Raumes durch Luftionisation unter Durchführung des Verfahrens nach den Patentansprüchen 1 bis 6, wobei an den wenigstens einen mit behandelter Luft zu versehenen Raum eine behandelte Luft zuführende Zuluftleitung angeschlossen ist, **dadurch gekennzeichnet,**
**dass** sich im Raum (9) oder in einer Abluftleitung (10) des Raumes (9) oder in einer Umluftleitung (12) zwischen einer Abluftleitung (10) und dem Luftaufbereitungsgerät (3) der zweite Luftqualitätssensor (13) befindet,
**dass** die Zuluftleitung (4) zwischen dem Luftaufbereitungsgerät (3) und dem wenigstens einem Raum (9) mit mindestens einem Ionisationsapparat (5), einem Luftströmungsfühler (6), einem Luftfeuchtefühler (7) und einem Ozonsensor (8) versehen sind und dass die Luftqualitätssensoren (2, 13), der Luftströmungsfühler (6), der Luftfeuchtefühler (7) und der Ozonsensor (8) über Signalleitungen funktionell mit dem elektronischen Steuergerät (14) verbunden sind.

9. Vorrichtung nach Patentanspruch 8, **dadurch gekennzeichnet, dass** ein elektronisches Steuergerät (14) für eine zeitlich zugeschaltete periodische Wechselspannung als Wechselimpulse (15), Wechselimpulsrate und/oder Paket von Wechselimpulsen (15) bestimmter Anzahl mit dem Ionisationsapparat (5) verbunden ist.

10. Vorrichtung nach Patentanspruch 8 oder 9, **dadurch gekennzeichnet, dass** ein entsprechend der Höhe des Gehalts an Ozon ein elektrisches Signal oder ein entsprechend eines vorbestimmbaren Wertes oder mehrerer vorbestimmbarer Werte des Gehalts an Ozon ein elektrisches Signal abgebender Ozonsensor (8) mit der elektrischen Steuerung (16) verbunden ist.

## Claims

1. Method for air conditioning of at least one room by air ionisation, whereby ionisation is performed by electric discharge in ionisation tubes or in corona-discharge tubes, whereby the level of the ionisation output is determined by an electronic controller (14) dependent on values of
- oxidizable air constituents in the air to be conditioned,
- relative air humidity of the air to be conditioned,
- flow velocity or volume flow rate of the air to be conditioned,
- ozone load on the inlet air, and
- minimum intensity of oxygen ions,
determined by sensors, namely a first air quality sensor (2), an air flow sensor (6), an air humidity sensor (7), an ozone sensor (8) and a second air quality sensor (13).

2. Method to claim 1, **characterized by that**
- particularly, the load on the outside air by volatile carbon hydroxides is measured using a first air quality sensor (2),
- the flow velocity or the volume flow rate of the air to be conditioned is measured using an air flow sensor (6),
- the relative air humidity of the air to be conditioned is measured using an air humidity sensor (7),
- the ozone content of the inlet air and/or the air to be conditioned is measured using an ozone sensor (8), and
- the oxidizable air constituents of the room (9) or of the outlet air of the room (9) in an outlet air conduit (10) or of the recirculation air in a recirculation air conduit (12) between an outlet air conduit (10) and an air conditioning device (3) is measured using the second air quality sensor (13),
and according to the measurement values, the level of the ionisation output of at least one or several ionisation apparatuses (5) is regulated by an electronic controller (14) such that the minimum intensity of oxygen ions is ensured and for too high an ozone value, the ozone value is reduced by breakdown.

3. Method to claim 1 or 2, **characterized by** that a minimum intensity of oxygen ions of approximately 5 % of the ionisation capacity installed is ensured.

4. Method to any of the claims 1 to 3, **characterized by** that the ionisation output is increased by logical combination of the measured values for a higher proportion of volatile carbon hydroxides and/or of the air velocity and/or of the relative air humidity and/or of the oxidizable air constituents.

5. Method to any of the claims 1 to 4, **characterized by** that the ionisation apparatus (5) is controllable periodic alternating voltage applied over a time period as at least one alternating voltage pulse (15) or at least one packet with a determined succession of alternating voltage pulses (15).

6. Method to any of the claims 1 to 5, **characterized by** that for an ozone content greater than or equal to 0.06 ppm in the inlet air conduit (4), the output of the ionisation apparatus (5) is reduced and that for a continued increase of the ozone value the time period of the periodic alternating voltage as alternating voltage pulses (15) and/or packets with alternating voltage pulses (15) of a determined number is changed such that breakdown of the ozone is initiated in the ionisation apparatus (5) which is connected to the ozone sensor (8) over the electronic controller (14).

7. Method to any of the claims 1 to 6, **characterized by** that for low process data causing no change of the electronic controller (14), an ionisation intensity exists.

8. Device for air conditioning of at least one room by air ionisation carrying out the method according to the claims 1 to 6, whereby an inlet air conduit supplying conditioned air is connected to the at least one room to be provided with conditioned air, **characterized by** that
in room (9) or in an air outlet conduit (10) of the room (9) or in a recirculation air conduit (12), between an air outlet conduit (10) and the air conditioning device (3), the second air quality sensor (13) is provided,
the inlet air conduit (4) between the air conditioning device (3) and the at least one room (9) is provided with at least one ionisation apparatus (5), an air flow sensor (6), an air humidity sensor (7) and an ozone sensor (8), and the air quality sensors (2, 13), the air flow sensor (6), the air humidity sensor (7) and the ozone sensor (8) are functionally connected to the electronic controller (14) over signal lines.

9. Device to claim 8, **characterized by** that an electronic controller (14) for a periodic alternating voltage switched on for a period of time as alternating voltage pulses (15), alternating voltage pulse rate and/or packet of alternating voltage pulses (15) of a determined number is connected to the ionisation apparatus (5).

10. Device to claim 8 or 9, **characterized by** that an ozone sensor (8) delivering an electric signal according to the level of the ozone content or delivering an electric signal according to a predeterminable value or several predeterminable values of the ozone content is connected to the electric control (16).

## Revendications

1. Procédé pour le traitement de l'air d'au moins une pièce par ionisation de l'air, cette ionisation étant réalisée par décharge électrique dans des tubes à ionisation ou dans des tubes à décharge en couronne ou par effet de couronne, la valeur ou niveau de la puissance d'ionisation étant déterminée par un appareil de commande électronique (14) en fonction des valeurs
- des constituants de l'air oxydables dans l'air à traiter,
- de l'humidité relative de l'air à traiter,
- de la vitesse d'écoulement ou du débit volumique de l'air à traiter,
- de la charge en ozone de l'air amené et
- de l'intensité minimale en ions oxygène
déterminées par des capteurs, à savoir un premier capteur de qualité d'air (2), un détecteur de flux ou débit d'air (6), un détecteur d'humidité de l'air (7), un capteur d'ozone (8) et un second capteur de qualité d'air (13).

2. Procédé selon la revendication 1, **caractérisé par le fait que**
- en particulier la charge de l'air extérieur en hydrocarbure volatile est mesurée avec un premier capteur de qualité d'air (2),
- la vitesse d'écoulement ou le débit volumique de l'air à traiter avec un détecteur de débit d'air (6),
- l'humidité relative de l'air à traiter avec un détecteur d'humidité de l'air (7),
- la teneur en ozone de l'air amené et/ou de l'air à traiter avec un capteur d'ozone (8) et
- les constituants de l'air oxydables de la pièce (9) ou l'air évacué de la pièce (9) d'une conduite d'évacuation d'air (10) ou l'air recirculé dans une conduite de recirculation d'air (12) entre une conduite d'évacuation (10) et un appareil de traitement d'air (3) avec le second capteur de qualité d'air (13) et
la valeur de la puissance d'ionisation d'au moins un ou plusieurs appareils d'ionisation (5) est régulée en fonction des valeurs des mesures par un appareil de commande électronique (14) de manière que l'intensité minimale en ions oxygène soit réduite et, en cas d'apparition d'une valeur d'ozone trop élevée, la valeur d'ozone soit réduite par dissociation.

3. Procédé selon la revendication 1 ou 2, **caractérisé par le fait qu'**une intensité minimale en ions oxygène d'environ 5 % de la capacité d'ionisation installée est garantie.

4. Procédé selon l'une des revendications 1 à 3, **caractérisé par le fait que** la puissance d'ionisation augmente par combinaison logique des valeurs mesurées en cas de d'augmentation de la proportion d'hydrocarbures volatils et/ou de la vitesse d'air et/ou de l'humidité relative de l'air et/ou de la proportion de constituants de l'air oxydables.

5. Procédé selon chaque fois l'une des revendications 1 à 4, **caractérisé par le fait que** l'appareil d'ionisation (5) peut être commandé par une tension alternative périodique appliquée temporairement sous la forme d'au moins une impulsion alternative (15) ou d'au moins un paquet comportant une suite définie d'impulsions alternatives (15).

6. Procédé selon l'une des revendications 1 à 5, **caractérisé par le fait qu'**en cas de teneur en ozone supérieure ou égale à 0,06 ppm dans la conduite d'amenée d'air (4), la puissance de l'appareil d'ionisation (5) est diminuée et qu'en cas de nouvelle augmentation de la valeur d'ozone le temps de la tension alternative périodique appliquée sous la forme d'impulsions alternatives (15) et/ou de paquets d'impulsions alternatives (15) en nombre défini est changé de manière qu'une dissociation de l'ozone soit initiée dans l'appareil d'ionisation (5) relié au capteur d'ozone (8) par l'intermédiaire de l'appareil de commande électronique (14).

7. Procédé selon chaque fois l'une des revendications 1 à 4, **caractérisé par le fait qu'**en cas de données de processus basses ou faibles et ne provoquant pas de changement de l'appareil de commande électronique (14), une intensité d'ionisation est présente.

8. Dispositif pour le traitement de l'air d'au moins une pièce par ionisation de l'air en réalisant le procédé selon les revendications 1 à 6, au moins une conduite d'amenée d'air amenant de l'air traité étant raccordée à ladite au moins une pièce à pourvoir en air traité, **caractérisé par le fait**
**que** le second capteur de qualité d'air (13) se trouve dans la pièce (9) ou dans une conduite d'évacuation d'air (10) de la pièce (9) ou dans une conduite de recirculation d'air (12) entre une conduite d'évacuation d'air (10) et l'appareil de traitement d'air (3),
**que** la conduite d'amenée d'air (4) entre l'appareil de traitement d'air (3) et ladite au moins une pièce (9) est munie d'au moins un appareil d'ionisation (5), d'un détecteur de débit d'air (6), d'un détecteur d'humidité de l'air (7) et d'un capteur d'ozone (8) et que les capteurs de qualité d'air (2, 13), le détecteur de débit d'air (6), le détecteur d'humidité de l'air (7) et le capteur d'ozone (8) sont reliés fonctionnellement à l'appareil de commande électronique (14) par des lignes de signaux.

9. Dispositif selon la revendication 8, **caractérisé par le fait qu'**un appareil de commande électronique (14) pour une tension alternative périodique activée temporairement sous forme d'impulsions alternatives (15), de taux d'impulsions alternatives et/ou de paquets d'impulsions alternatives (15) en nombre défini est relié à l'appareil d'ionisation (5).

10. Dispositif selon la revendication 8 ou 9, **caractérisé par le fait qu'**un capteur d'ozone (8) délivrant un signal électrique correspondant à la valeur de la teneur en ozone ou un signal électrique correspondant à une valeur prédéterminable ou à plusieurs valeurs prédéterminables de la teneur en ozone est relié à la commande électrique (16).
